# EUROPEAN PATENT APPLICATION

(11) **EP 4 684 787 A1**
(43) Date of publication of application: **28.01.2026**
(21) Application number: 24774258.8
(22) Date of filing: 22.03.2024
(51) Int. Cl.: A61K 31/553, A61P 11/06

(54) **PHARMACEUTICAL COMPOSITION CONTAINING DIPEPTIDYL PEPTIDASE SMALL MOLECULE INHIBITOR**

(30) Priority: 23.03.2023 CN 202310291119
(71) Applicant: Haisco Pharmaceuticals Pte. Ltd., Singapore 079903 (SG)
(72) Inventor: XU, Zaiyang, Lhoka, Tibet 856099 (CN); CUI, Xiaofei, Lhoka, Tibet 856099 (CN); REN, Dong, Lhoka, Tibet 856099 (CN); LIU, Bo, Lhoka, Tibet 856099 (CN); YAO, Zhi´ang, Lhoka, Tibet 856099 (CN)
(74) Representative: Bianchetti & Minoja with Trevisan & Cuonzo IPS SRL
(86) International application number: PCT/CN2024/083306
(87) International publication number: WO 2024/193695

(57) **Abstract**

A pharmaceutical composition comprising a Dipeptidyl Peptidase 1 (DPP1) small molecule inhibitor, Compound A, and a preparation method thereof, and a use of the pharmaceutical composition in the treatment of diseases or conditions associated with fibrotic bronchiectasis.

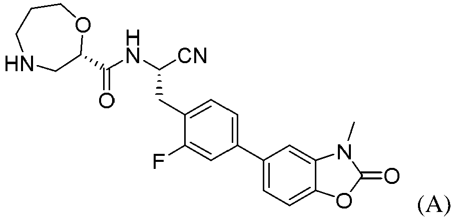

## Description

### TECHNICAL FIELD

The present invention relates to the technical field of a formulation comprising a chemical drug, and particularly to a pharmaceutical composition comprising a dipeptidyl peptidase small molecule inhibitor Compound A for treating diseases associated with fibrotic bronchiectasis, as well as a preparation thereof.

### BACKGROUND

Non-cystic fibrotic bronchiectasis is a disease wherein recurrent purulent infections caused by various etiological factors lead to repeated damage to and/or obstruction of medium and small bronchi, thereby damaging the structure of the bronchial wall and causing abnormal and persistent dilation of the bronchi. Its clinical manifestations vary in severity, including chronic cough, excessive expectoration, and/or intermittent hemoptysis, with or without short breath and respiratory failure. All factors that cause damage to the structure of the bronchial wall, including infection, immunodeficiency, rheumatism, bronchial asthma, chronic obstructive pulmonary disease, etc., may cause bronchiectasis. The most common and typical symptoms are chronic cough and expectoration associated with mucopurulent secretions. With the progression of the disease and/or deterioration of lung function, such symptoms as dyspnea, fatigue and hemoptysis may also occur. In recent years, an increased incidence and prevalence of bronchiectasis has been reported internationally. According to statistics, the incidence and the prevalence of bronchiectasis among the British increased to 31.1/100,000 and 525.8/100,000 by 2013, respectively; the incidence of bronchiectasis among Spaniards was approximately 48.1/100,000 in 2012; and the prevalence of bronchiectasis among US adults was about 139/100,000.

Acute lung injury / acute respiratory distress syndrome is an acute hypoxic respiratory insufficiency or failure resulting from diffuse pulmonary interstitial and alveolar edema, which arises from damage to pulmonary capillary endothelial cells and alveolar epithelial cells during non-cardiogenic conditions such as severe infection, shock, trauma, and burns. Its pathophysiological characteristics include reduced lung volume, decreased lung compliance, and severe ventilation/perfusion imbalance. The clinical manifestations include progressive hypoxemia and respiratory distress, and lung imaging shows heterogeneous exudative lesions. Due to different definitions and the heterogeneity of the disease, it is difficult to accurately determine the incidence and mortality of ARDS. A review by Rubenfeld and Herridge in 2007 reported that the incidence of ARDS ranges from 13.5 to 58.7/100,000 people/year, and the mortality ranges from approximately 34% to 57.9%.

Compound A, disclosed in WO2022042591A1, is a potent and highly selective small-molecule inhibitor of Dipeptidyl Peptidase (Dipeptidyl Peptidase 1, DPP1). It has been used in the treatment of lower respiratory tract diseases caused by bronchiectasis (including non-cystic fibrotic bronchiectasis and cystic fibrotic bronchiectasis) and acute lung injury/acute respiratory distress syndrome. Preclinical studies showed that Compound A can significantly inhibit NE enzyme activity in rat bone marrow and mitigate LPS-induced acute lung injury in mice, exhibiting good pharmacological efficacy.

### DISCLOSURE OF THE INVENTION

The object of the present invention is to provide a pharmaceutical composition of Compound A, which comprises Compound A and pharmaceutically acceptable excipients. The composition of the present invention exhibits consistent quality, good stability, high bioavailability, and rapid dissolution. When formulated as an immediate-release tablet, the composition has the advantages of consistent quality, precise dosage, convenient transportation, cost-effective manufacturing, etc. Upon administration, the drug can be rapidly released and absorbed by the body.

The present invention provides a pharmaceutical composition comprising Compound A and pharmaceutically acceptable excipients. The structure of Compound A is shown below:

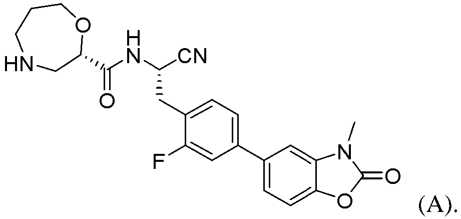

The present invention provides a pharmaceutical composition, wherein the pharmaceutically acceptable excipients include one or more of, for example, a filler, a disintegrant, a lubricant, an antioxidant, a binder, a flavoring agent, a coloring agent, a coating material, etc.

The present invention provides a pharmaceutical composition comprising Compound A and a filler.

The present invention provides a pharmaceutical composition comprising Compound A, a filler, and a disintegrant.

The present invention provides a pharmaceutical composition comprising Compound A, a filler, a disintegrant, and a lubricant.

The present invention provides a pharmaceutical composition comprising Compound A, a filler, a disintegrant, a lubricant, and a coating material.

The present invention provides an oral-administrated pharmaceutical composition comprising Compound A and pharmaceutically acceptable excipients, and specifically comprising: (1) Compound A, (2) a filler, (3) a lubricant, (4) a disintegrant, (5) a coating material, wherein Compound A has the structure of:

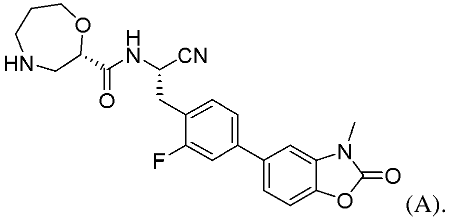

In certain embodiments, the amount of Compound A in the pharmaceutical composition of the present invention is 1%-40% by mass of the total composition. In certain embodiments, the amount of Compound A is 1%-20% by mass of the total composition. In certain embodiments, the amount of Compound A is 2.5%-10% by mass of the total composition. In certain embodiments, the amount of Compound A is 5%-10% by mass of the total composition. In certain embodiments, the amount of Compound A is 5% by mass of the total composition.

The present invention provides a pharmaceutical composition, wherein the particle size distribution d(0.9) of Compound A is less than or equal to 150 µm, preferably less than or equal to 100 µm, and more preferably less than or equal to 85 µm.

In the pharmaceutical composition of the present invention, the processing method for Compound A is one or more of sieving, mechanical grinding, and air-flow grinding.

In the pharmaceutical composition of the present invention, the filler may be selected from one or more of lactose, microcrystalline cellulose, sucrose, glucose, powdered cellulose, calcium phosphate, calcium hydrogen phosphate, calcium carbonate, aluminum silicate, dextrin, starch (including corn starch, potato starch or amylopectin), pregelatinized starch, sodium chloride, potassium chloride, mannitol, and sorbitol.

In certain embodiments, in the pharmaceutical composition of the present invention, the filler is selected from one or more of lactose, microcrystalline cellulose, and mannitol.

In certain embodiments, the amount of filler in the pharmaceutical composition of the present invention is from 67% to 99% by mass of the total composition. In certain embodiments, the amount of filler is from 70% to 99% by mass of the total composition. In certain embodiments, the amount of filler is from 80% to 90% by mass of the total composition. In certain embodiments, the amount of filler is 89% by mass of the total composition.

In certain embodiments, in the pharmaceutical composition of the present invention, the filler is mannitol and microcrystalline cellulose, and the mass ratio of mannitol to microcrystalline cellulose is from 5:1 to 1:1. In certain embodiments, the mass ratio of mannitol to microcrystalline cellulose is from 4:1 to 1:1. In certain embodiments, the mass ratio of mannitol to microcrystalline cellulose is from 3:1 to 1:1. In certain embodiments, the mass ratio of mannitol to microcrystalline cellulose is from 2:1 to 1:1. In certain embodiments, the mass ratio of mannitol to microcrystalline cellulose is 1:1.

In certain embodiments, in the pharmaceutical composition of the present invention, the filler is lactose and microcrystalline cellulose, and the mass ratio of lactose to microcrystalline cellulose is from 5:1 to 1:1. In certain embodiments, the mass ratio of lactose to microcrystalline cellulose is from 4:1 to 1:1. In certain embodiments, the mass ratio of lactose to microcrystalline cellulose is from 3:1 to 1:1. In certain embodiments, the mass ratio of lactose to microcrystalline cellulose is from 2:1 to 1:1. In certain embodiments, the mass ratio of lactose to microcrystalline cellulose is 3:1.

In the pharmaceutical composition of the present invention, the disintegrant may be selected from one or more of starch, pregelatinized starch, sodium carboxymethyl starch, cross-linked povidone and croscarmellose sodium.

In certain embodiments, the amount of disintegrant in the pharmaceutical composition of the present invention is from 0.5% to 10% by mass of the total composition. In certain embodiments, the amount of disintegrant is 1% to 10% by mass of the total composition. In certain embodiments, the amount of disintegrant is from 2% to 9% by mass of the total composition. In certain embodiments, the amount of disintegrant is from 3% to 8% by mass of the total composition. In certain embodiments, the amount of disintegrant is from 3% to 7% by mass of the total composition. In certain embodiments, the amount of disintegrant is 5% by mass of the total composition.

In certain embodiments, the disintegrant in the pharmaceutical composition of the present invention is cross-linked povidone.

In certain embodiments, the amount of cross-linked povidone in the pharmaceutical composition of the present invention is from 0.5% to 10% by mass of the total composition. In certain embodiments, the amount of cross-linked povidone is from 1% to 7% by mass of the total composition. In certain embodiments, the amount of cross-linked povidone is from 3% to 7% by mass of the total composition. In certain embodiments, the amount of cross-linked povidone is from 3% to 5% by mass of the total composition. In certain embodiments, the amount of cross-linked povidone in the pharmaceutical composition of the present invention is 5% by mass of the total composition.

In the pharmaceutical composition of the present invention, the lubricant may be selected from one or more of stearic acid, magnesium stearate, calcium stearate, aluminum stearate, palmitic acid, glyceryl behenate, polyethylene glycols with various molecular weights, hydrogenated castor oil or sodium stearyl fumarate.

In certain embodiments, the amount of lubricant in the pharmaceutical composition of the present invention is from 0.25% to 2% by mass of the total composition. In certain embodiments, the amount of lubricant is from 0.5% to 1.5% by mass of the total composition. In certain embodiments, the amount of lubricant is from 1% to 1.5% by mass of the total composition. In certain embodiments, the amount of lubricant is 1% by mass of the total composition.

In certain embodiments, the lubricant in the pharmaceutical composition of the present inventions is magnesium stearate.

In certain embodiments, the amount of magnesium stearate in the pharmaceutical composition of the present invention is from 0.25% to 2% by mass of the total composition. In certain embodiments, the amount of magnesium stearate is from 0.5% to 1.5% by mass of the total composition. In certain embodiments, the amount of magnesium stearate is from 1% to 1.5% by mass of the total composition. In certain embodiments, the amount of magnesium stearate is 1% by mass of the total composition.

In certain embodiments, the pharmaceutical composition of the present invention further comprises a binder. The binder may be selected from one or more of starch slurry, povidone, hydroxypropyl cellulose, hydroxypropyl methylcellulose, ethyl cellulose, sodium carboxymethyl cellulose, xanthan gum, gum arabic, gelatin, guar gum or carbomer.

In certain embodiments, the amount of binder in the pharmaceutical composition of the present invention is from 0.1% to 10% by mass of the total composition. In certain embodiments, the amount of binder is from 3% to 10% by mass of the total composition. In certain embodiments, the amount of binder is from 3% to 7% by mass of the total composition. In certain embodiments, the amount of binder is 7% by mass of the total composition.

In certain embodiments, the binder in the pharmaceutical composition of the present invention is selected from povidone.

In certain embodiments, the binder in the pharmaceutical composition of the present invention is selected from povidone, and the amount thereof is from 0.1% to 10% by mass of the total composition. In certain embodiments, the amount of povidone is from 3% to 10% by mass of the total composition. In certain embodiments, the amount of povidone is from 3% to 7% by mass of the total composition. In certain embodiments, the amount of povidone is 7% by mass of the total composition.

In certain embodiments, the pharmaceutical composition of the present invention further comprises a coating material. The coating material may be selected from a film coating premix (gastric soluble) or a mixture of one or more of the following coating materials: polyvinyl alcohol, hydroxypropyl methylcellulose, talc, polyethylene glycol, polyethylene glycol-polyvinyl alcohol copolymer, titanium dioxide, glyceryl mono- and dicaprylocaprate, and lake.

In certain embodiments, the target coating weight gain in the pharmaceutical composition of the present invention is from 0.5% to 10% by mass of the tablet core. In certain embodiments, the target coating weight gain is from 1% to 6% by mass of the tablet core. In certain embodiments, the target coating weight gain is from 2% to 4% by mass of the tablet core.

In certain embodiments, the coating material in the pharmaceutical composition of the present invention is selected from a film coating premix (gastric soluble).

In certain embodiments, the coating material in the pharmaceutical composition of the present invention is selected from a film coating premix (gastric soluble), and the target coating weight gain is from 0.5% to 10% by mass of the tablet core. In certain embodiments, the target coating weight gain is from 1% to 6% by mass of the tablet core. In certain embodiments, the target coating weight gain is from 2% to 4% by mass of the tablet core.

The pharmaceutical composition of the present invention may be prepared into tablets, capsules, granules or powders.

The present invention further relates to a pharmaceutical composition comprising (1) Compound A, (2) a filler, (3) a lubricant, (4) a disintegrant, and (5) a coating material.

The present invention further relates to a pharmaceutical composition comprising:
(1) Compound A in an amount of 1%-20%, preferably 2.5%-10%, more preferably 5%, by mass of the total composition;
(2) a filler in an amount of 70% to 99%, preferably 80% to 90%, more preferably 89%, by mass of the total composition;
(3) a lubricant in an amount of 0.25% to 2%, preferably 0.5% to 1.5%, more preferably 1%, by mass of the total composition;
(4) a disintegrant in an amount of 0.5% to 10%, preferably 3% to 7%, more preferably 5%, by mass of the total composition;
(5) a coating material, and the target coating weight gain is 0.5% to 10%, preferably 1% to 6%, more preferably 2% to 4%, by mass of the tablet core.

The present invention further relates to a pharmaceutical composition comprising:
(1) Compound A in an amount of 1%-20%, preferably 2.5%-10%, more preferably 5%, by mass of the total composition;
(2) a filler, wherein the filler is mannitol and microcrystalline cellulose in a mass ratio of 3:1 to 1:1, preferably 1:1; the amount of filler is 70% to 99%, preferably 80% to 90%, more preferably 89%, by mass of the total composition;
(3) a lubricant, wherein the lubricant is magnesium stearate, in an amount of 0.25% to 2%, preferably 0.5% to 1.5%, more preferably 1%, by mass of the total composition;
(4) a disintegrant, wherein the disintegrant is cross-linked povidone, in an amount of 0.5% to 10%, preferably 3% to 7%, more preferably 5%, by mass of the total composition;
(5) a coating material, wherein the coating powder is a film coating premix (gastric soluble), and the target coating weight gain is 0.5% to 10%, preferably 1% to 6%, more preferably 2% to 4%, by mass of the tablet core.

In certain embodiments, the unit dosage of the pharmaceutical composition formulation of the present invention is selected from 1 mg to 100 mg, preferably 1 mg, 5 mg, 10 mg, 15 mg, 20 mg, 25 mg, 30 mg, 40 mg, 50 mg, 60 mg, 75 mg, 100 mg, calculated based on the active Compound A.

In certain embodiments, the pharmaceutical composition of the present invention is tablet.

In certain embodiments, in the pharmaceutical composition of the present invention, the dosage in each unit formulation of the tablet is selected from 1mg - 100 mg, preferably 1 mg, 5 mg, 10 mg, 15 mg, 20 mg, 25 mg, 30 mg, 40 mg, 50 mg, 60 mg, 75 mg, 100 mg, calculated based on the active Compound A.

In the pharmaceutical composition of the present invention, the active substance Compound A is present in an amount of 1-100 mg. In some embodiments, the active substance Compound A is present in an amount of 2-80 mg; in some embodiments, in an amount of 2-60 mg; in some embodiments, in an amount of 2-50 mg; in some embodiments, in an amount of 5-50 mg; in some embodiments, in an amount of 5-40 mg; in some embodiments, in an amount of 5-30 mg; in some embodiments, in an amount of 5-20 mg; in some embodiments, in an amount of 5-10 mg; in some embodiments, in an amount of 10-80 mg; in some embodiments, in an amount of 10-60 mg; in some embodiments, in an amount of 10-50 mg; in some embodiments, in an amount of 20-80 mg; in some embodiments, in an amount of 20-60 mg; in some embodiments, in an amount of 20-50 mg; and in some embodiments, in an amount of 30-50 mg.

The pharmaceutical composition of the present invention is tablet or capsule, and the active substance is present in an amount of 5 mg, 10 mg, 15 mg, 20 mg, 25 mg, 30 mg, 35 mg, 40 mg, 45 mg or 50 mg. In certain embodiments, the active substance is present in an amount of 5 mg, 10 mg or 20 mg. In certain embodiments, the active substance is present in an amount of 5 mg or 20 mg.

The present invention also provides a preparation method of the formulation of the pharmaceutical composition, including one or more of dry granulation process, wet granulation process, and direct powder compression process.

In certain embodiments, the unit formulation of the pharmaceutical composition of the present invention comprises 5 mg Compound A, 44.5 mg mannitol, 44.5 mg microcrystalline cellulose, 3 mg coating powder, 5 mg cross-linked povidone, 1 mg magnesium stearate, or the unit formulation of the pharmaceutical composition comprises 20 mg Compound A, 178 mg mannitol, 178 mg microcrystalline cellulose, 20 mg cross-linked povidone, 4 mg magnesium stearate, and 12 mg coating powder.

The present invention also provides a method for preparing the formulation of the pharmaceutical composition, comprising the steps of:
(1) crushing Compound A;
(2) adding Compound A, a filler and a disintegrant into a mixer for premixing;
(3) adding a lubricant into the mixer and mixing thoroughly with the premixed powder; the mixture is compressed into a tablet, film-coated, and packaged.

The mixer equipment includes but is not limited to one or more of: a mixer which mixes the materials by driving stirring blades or cutting knives at high speed, such as a wet mixing granulator or a high-energy mixer, or a mixer which mixes the materials in a container by rotating the equipment container, such as a three-dimensional mixer or a hopper mixer.

In the drug preparation process of the present invention, a wet mixing granulator is employed to premix the starting or auxiliary materials, which optimizes the mixing process, reduces the mixing steps of traditional mixing process, such as equal increment addition and multi-step mixing, reduces the time for mixing, has a high mixing efficiency, and has a superior material mixing uniformity to the conventional mixing methods.

The present invention also provides a use of the pharmaceutical composition in the manufacture of a medicament for treating diseases associated with fibrotic bronchiectasis, wherein the diseases associated with fibrotic bronchiectasis are selected from bronchiectasis (including non-cystic fibrotic bronchiectasis and cystic fibrotic bronchiectasis), and lower respiratory tract diseases caused by acute lung injury/acute respiratory distress syndrome.

Unless otherwise specified, the expression "amount" of each ingredient in the present invention refers to the percentage by mass of the substance in the total mass of the composition. For such formulations as tablets or capsules, the percentage by weight of each ingredient is the percentage by mass of the ingredient in the tablet or capsule contents (including the active agent and the excipients).

The pharmaceutical composition formulation provided in the present invention has good stability and bioavailability.

### Embodiments

The present invention is further illustrated in detail by the following examples. Where no specific conditions are described, the examples were carried out according to conventional conditions of the experimental method. The examples are given to better illustrate the contents of the invention, and it shall not be understood that the contents of the present invention are limited by the examples. According to the above disclosure of the invention, those skilled in the art can modify and adjust the embodiments without departing from the spirit of the present invention. These modified or adjusted technical solutions still fall within the protection scope of the present invention.

Unless otherwise specified, the materials used in the examples of the present invention are all commercially available.

### Preparation of Compound A:

### (S)-N-((S)-1-cyano-2-(2-fluoro-4-(3-methyl-2-oxo-2,3-dihydrobenzo[d]oxazol-5-yl)phenyl)ethyl)-1,4-oxazepane-2-carboxamide (Compound A)

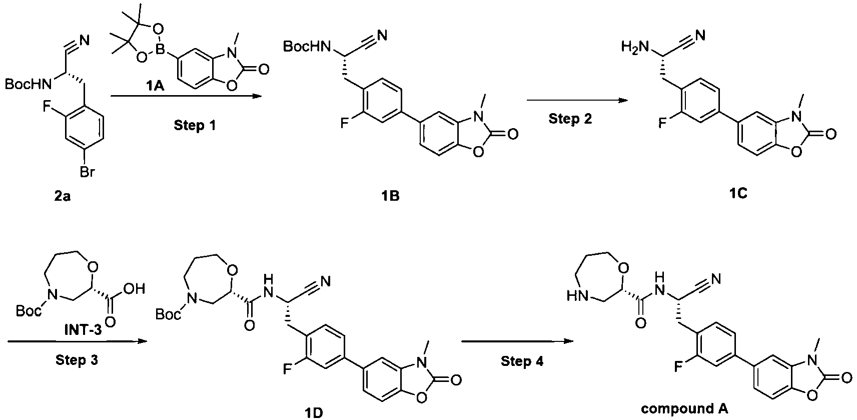

### Step 1: (S)-tert-butyl (1-cyano-2-(2-fluoro-4-(3-methyl-2-oxo-2,3-dihydrobenzo[d]oxazol-5-yl)phenyl)ethyl)carbamate (1B)

1A (0.29 g, 0.85 mmol, synthesized by reference to WO 2016016242 A1) was dissolved in 1,4-dioxane (10 mL) and water (0.4 mL). The intermediate 2a (0.35 g, 1.27 mmol, synthesized by reference to WO 2016016242 A1), potassium carbonate (0.24 g, 1.70 mmol), and [1,1'-bis(diphenylphosphino)ferrocene]palladium dichloride dichloromethane complex (70 mg, 0.09 mmol) were added. Upon completion of the addition, the mixture was reacted at 90° C for 3 h. The reaction was cooled to room temperature, and saturated aqueous sodium chloride solution (20 mL) was added. The resulting mixture was extracted with ethyl acetate (20 mL×3). The organic phases were combined, dried over anhydrous sodium sulfate, filtered and concentrated. The residue was purified by silica gel column chromatography (PE:EA (v/v)=4:1) to obtain the title compound 1B (white solid, 0.34 g, 99.0%).

LC-MS (ESI): m/z =412.1 [M+H]⁺.

### Step 2: (S)-2-amino-3-(2-fluoro-4-(3-methyl-2-oxo-2,3-dihydrobenzo[d]oxazol- 5-yl)phenyl)propanenitrile (1C)

1B (0.34 g, 0.83 mmol) was dissolved in formic acid (5 mL). Upon completion of the addition, the mixture was reacted at room temperature overnight. The reaction was concentrated to dryness, and ethyl acetate (25 mL) was added. Then, saturated aqueous sodium bicarbonate solution was added dropwise to adjust the pH to about 8. The organic layer was separated and the aqueous layer was extracted with ethyl acetate (25 mL×3). The organic phases were combined, dried over anhydrous sodium sulfate, filtered and concentrated to obtain a crude product of the title compound 1C (pale yellow solid, 0.21 g, 69.5%).

LC-MS (ESI): m/z =312.1 [M+H]⁺.

### Step 3: (S)-tert-butyl 2-(((S)-1-cyano-2-(2-fluoro-4-(3-methyl-2-oxo-2,3-dihydrobenzo[d]oxazol-5-yl)phenyl)ethyl)carbamoyl)-1,4-oxazepane-4-carboxylate (1D)

1C (0.21 g, 0.60 mmol) was dissolved in DMF (10 mL), and DIPEA (0.23 g, 1.80 mmol), HATU (0.34 g, 0.90 mmol), and INT-3 (0.22 g, 0.90 mmol, prepared by reference to WO 2015110826) were added. Upon completion of the addition, the mixture was reacted at room temperature overnight. The reaction was quenched by adding saturated aqueous ammonium chloride solution dropwise, and saturated aqueous sodium chloride solution (30 mL) was added. The mixture was extracted with ethyl acetate (25 mL). The organic phase was washed with saturated aqueous sodium chloride solution (25 mL×3), dried over anhydrous sodium sulfate, filtered and concentrated to obtain the title compound 1D (pale yellow solid, 0.32 g, 99.0% yield), which was directly used in the next step.

LC-MS (ESI): m/z =483.1 [M-57+H]⁺.

### Step 4: (S)-N-((S)-1-cyano-2-(2-fluoro-4-(3-methyl-2-oxo-2,3-dihydrobenzo[d]oxazol-5-yl)phenyl)ethyl)-1,4-oxazepane-2-carboxamide (Compound A)

1D (0.32 g, 0.59 mmol) was dissolved in formic acid (2.5 mL). Upon completion of the addition, the mixture was reacted at 50° C for 10 min. The reaction was concentrated to dryness, and ethyl acetate (20 mL) was added. Then, saturated aqueous sodium bicarbonate solution was added dropwise to adjust the pH to about 8. The organic layer was separated and the remaining aqueous layer was extracted with ethyl acetate (25 mL×3). The organic phases were combined, dried over anhydrous sodium sulfate, filtered and concentrated. The residue was purified by silica gel column chromatography (dichloromethane : methanol (v/v)=20:1) to obtain the title compound 1 (0.15 g, 58.0% yield).

LC-MS (ESI): m/z =439.1 [M+H]⁺.

¹H NMR (400 MHz, CDCl₃) δ 7.43 - 7.22 (m, 5H) 7.12 (d, 1H) 5.19 (dd, 1H) 4.18 - 4.04 (m, 1H) 4.05 - 3.95 (m, 1H) 3.78 (m, 1H) 3.46 (s, 3H) 3.41 - 3.17 (m, 3H) 3.03 - 2.87 (m, 3H) 1.88 (m, 2H).

### Example 1-3

The formulations of Examples 1-3 are shown in Table 1.

The preparation steps are as follows:
(1) Compound A was mechanically grinded.
(2) Compound A, lactose, microcrystalline cellulose, povidone and cross-linked povidone were added into the wet mixing granulator, and the stirrer (500 rpm) and shearing blades (2000 rpm) were turned on to mix the materials thoroughly.
(3) With the stirrer and shearing blades turned on, an appropriate amount of purified water was added to the granulator to prepare a suitable soft material, which was granulated through a 20-mesh sieve; the wet granules were dried below 60°C.
(4) The dried granules were sieved through a 20-mesh screen, then blended with magnesium stearate until uniform. The materials were compressed into a tablet using a 6.5 mm round punch to obtain uncoated tablets.
(5) Water was added to the film coating premix (gastric soluble) from Colorcon under stirring to prepare a coating solution with a solid content of 30%. The uncoated tablets were placed in a high-efficiency coating machine and the coating solution was sprayed to coat the tablets. The target coating weight gain is 2% to 4% by weight of the table core. The film-coated tablets were obtained.

**Table 1 Composition and dissolution of Examples 1 to 3**

| Items | | Example 1 | Example 2 | Example 3 |
|---|---|---|---|---|
| Compositions of uncoated tablets (mg per tablet) | Compound A | 5 | 5 | 5 |
| | Lactose | 62 | 60 | 58 |
| | Microcrystalline cellulose | 22 | 22 | 22 |
| | Povidone | 7 | 7 | 7 |
| | Cross-linked povidone | 3 | 5 | 7 |
| | Magnesium stearate | 1 | 1 | 1 |
| | Purified water | q.s. | q.s. | q.s. |
| Dissolution in pH6.8 buffer (%) | 5 min | 73.8 | 72.7 | 84.3 |
| | 10 min | 85.5 | 87.7 | 95.7 |
| | 15 min | 88.7 | 90.6 | 98.4 |
| | 30 min | 90.3 | 92.7 | 99.5 |
| | 60 min | 94.1 | 97.5 | 103.1 |

Conclusion: with 3%-7% cross-linked polyvinylpyrrolidone as a disintegrant, the product can be rapidly disintegrated, and achieve a nearly complete dissolution.

### Examples 4 and 5

The formulations of Examples 4-5 are shown in Table 2.

The preparation steps are the same to those in Examples 1 to 3.

**Table 2 Composition and dissolution of Examples 4 and 5**

| Items | | Example 4 | Example 5 |
|---|---|---|---|
| Compositions (mg per tablet) | Compound A | 5 | 5 |
| | lactose | 60.5 | 59.5 |
| | Microcrystalline cellulose | 22 | 22 |
| | Povidone | 7 | 7 |
| | Cross-linked Povidone | 5 | 5 |
| | Magnesium stearate | 0.5 | 1.5 |
| | Purified water | q.s. | q.s. |
| Dissolution in pH6.8 buffer (%) | 5min | 81.1 | 69.2 |
| | 10min | 94.2 | 77.0 |
| | 15min | 97.8 | 79.8 |
| | 30min | 98.8 | 83.1 |
| | 60min | 101.0 | 86.7 |

Conclusion: combining with the dissolution results shown in Example 2, when 0.5% to 1.5% magnesium stearate was used, the products can all be rapidly disintegrated and dissolved, and exhibit sufficient lubricating characteristics during the production process.

### Example 6-8

The formulations of Examples 6-8 are shown in Table 3.

The preparation steps are as follows:
(1) Compound A was mechanically grinded.
(2) Compound A, mannitol, microcrystalline cellulose and cross-linked povidone were added to the wet mixing granulator, and the stirrer and shearing blades were turned on to mix the materials thoroughly.
(3) The above mixture was added into a hopper mixer. Magnesium stearate was added and mixed thoroughly. The mixed materials were compressed into tablets to obtain the product.

**Table 3. Composition and content uniformity of tablets of Examples 6 to 8**

| Items | | Example 6 | Example 7 | Example 8 |
|---|---|---|---|---|
| Compositions (mg per a tablet) | Compound A | 5 | 5 | 5 |
| | mannitol | / | 65.5 | 44.5 |
| | microcrystalline cellulose | 89 | 23.5 | 44.5 |
| | cross-linked povidone | 5 | 5 | 5 |
| | magnesium stearate | 1 | 1 | 1 |
| Relative standard deviation of tablet content (%) | | 0.79 | 1.02 | 1.02 |

Conclusion: when microcrystalline cellulose alone or a combination of mannitol and microcrystalline cellulose in a ratio of about 3:1 to 1:1 was used, the tablets have uniform content, good compressibility and consistent quality.

### Example 9

Compound A was mixed uniformly with lactose, microcrystalline cellulose and mannitol respectively at a ratio of 1:5. The mixture was placed in a wide-mouth glass container of appropriate size.

The above mixtures were respectively placed under the following conditions: 60°C closed, 92.5% RH (relative humidity) open, 40°C/75% RH closed, and 40°C/75% RH open. The change of impurities was measured after 2 weeks placement.

**Table 4. Stability of compound A mixed with different fillers**

| Compositions | Conditions | Maximum of single impurity | Total impurity |
|---|---|---|---|
| Compound A + mannitol | Day 0 | 0.036% | 0.208% |
| | 40°C/75% RH, closed, 2 weeks | 0.041% | 0.226% |
| | 40°C/75% RH, open, 2 weeks | 0.042% | 0.265% |
| | 60°C, closed, 2 weeks | 0.040% | 0.206% |
| | 92.5%RH, open, 2 weeks | 0.042% | 0.263% |
| Compound A + microcrystalline cellulose | Day 0 | 0.049% | 0.236% |
| | 40°C/75%RH, closed, 2 weeks | 0.046% | 0.183% |
| | 40°C/75%RH, open, 2 weeks | 0.045% | 0.269% |
| | 60°C, closed, 2 weeks | 0.046% | 0.241% |
| | 92.5%RH, open, 2 weeks | 0.044% | 0.229% |
| Compound A + lactose | Day 0 | 0.051% | 0.304% |
| | 40°C/75%RH, closed, 2 weeks | 0.067% | 0.295% |
| | 40°C/75%RH, open, 2 weeks | 0.094% | 0.385% |
| | 60°C, closed, 2 weeks | 0.079% | 0.328% |
| | 92.5%RH, open, 2 weeks | 0.102% | 0.366% |

Conclusion: according to the results after 2-weeks placement, Compound A keeps good stability after being mixed with various fillers, wherein mixing Compound A with mannitol or microcrystalline cellulose results in less impurity growth and superior stability.

### Example 10

The composition of the formulation of Example 10 is shown in Table 5.

The preparation steps are the same with those in Examples 6 to 8.

**Table 5. Composition of Example 10**

| Items | | Example 8 | Example 10 |
|---|---|---|---|
| | Compound A | 5 | 20 |
| | Mannitol | 44.5 | 178.0 |
| Compositions (mg per a tablet) | Microcrystalline cellulose | 44.5 | 178.0 |
| | cross-linked povidone | 5 | 20 |
| | Magnesium stearate | 1 | 4 |

Tablets with different hardness were prepared. They all have relatively complete dissolution, as shown in Table 6 below.

**Table 6. Dissolution of tablets with different hardness in Examples 8 and 10**

| Items | | Example 10 | | Example 8 | |
|---|---|---|---|---|---|
| Shape of tablet | | 15*7mm, oval tablet | | 6.5mm, round tablet | |
| Average hardness of tablet | | 140N | 80N | 55N | 25N |
| Dissolution in 0.1mol/L hydrochloric acid medium (%) | 5min | 92.3 | 91.7 | 90.7 | 89.5 |
| | 10min | 97.2 | 93.4 | 94.4 | 92.2 |
| | 15min | 98.9 | 96.2 | 94.5 | 93.2 |
| | 30min | 100.2 | 98.0 | 95.7 | 95.3 |
| | 60min | 101.6 | 99.2 | 97.1 | 96.4 |

Tablet hardness within the investigated range has no influence on the dissolution profile. The products have elegant appearance and good mechanical strength.

The products of Examples 8 and 10 were packaged in dual-aluminum blisters and placed in a test environment of 40°C, RH75% for 6 months. Compared with the results at 0 month, the dissolution and content at 6^{th} month keep unchanged, and the total impurities at 6^{th} month just increased slightly, suggesting good stability. The specific results are shown in Table 7 below.

**Table 7. Stability of Examples 8 and 10**

| Investigation Items | Example 8 | | Example 10 | |
|---|---|---|---|---|
| | 0 month | 6 months | 0 month | 6 months |
| Description | off-white tablet | off-white tablet | off-white tablet | off-white tablet |
| Total amount of impurity | 0.23% | 0.55% | 0.27% | 0.57% |
| Dissolution in buffer pH6.8, 30min | 97.7% | 99% | 93.0% | 96% |
| Content | 98.3% | 97.3% | 97.7% | 100.1% |

### Example 11-14

Compound A was processed into different particle sizes by different means. The composition of the formulations is the same as that in Example 10, and the preparation steps are as described in Examples 6 to 8. The effects of different particle sizes of Compound A on the mixing uniformity of the formulation and the dissolution profile of the tablet were investigated.

**Table 8. Content determination results**

| Examples | Processing methods for Compound A | Particle size d (0.9) of Compound A (µm) | Total mixed powder | | Tablet | |
|---|---|---|---|---|---|---|
| | | | Average content (%) | RSD (%) | Average content (%) | A+2.2S |
| Example 11 | Air-flow grinding | 7.349 | 4.99 | 1.2 | 98.97 | 3.07 |
| Example 12 | Mechanical grinding | 19.792 | 4.94 | 0.9 | 99.3 | 5.7 |
| Example 13 | Sieved | 82.334 | 4.98 | 1.3 | 97.57 | 4.77 |
| Example 14 | Unprocessed | 125.523 | 5.10 | 3.9 | 103.2 | 1.4 |

**Table 9. Dissolution profile results of tablets**

| Dissolution medium | Time (min) | Example 11 | Example 12 | Example 13 | Example 14 |
|---|---|---|---|---|---|
| | | Particle size d (0.9) of Compound A: 7.349µm | Particle size d (0.9) of Compound A: 19.792µm | Particle size d (0.9) of Compound A: 82.334µm | Particle size d (0.9) of Compound A: 125.523µm |
| | 5 | 55.6 | 57 | 58 | 39 |
| | 10 | 62.8 | 70 | 72 | 58 |
| pH6.8 buffer | 15 | 66.0 | 74 | 78 | 69 |
| | 30 | 70.3 | 79 | 84 | 81 |
| | 45 | 71.9 | 82 | 82 | 77 |
| | 60 | 73.3 | 57 | 84 | 80 |

The results showed that, where the particle size d(0.9) of the starting materials ranges from 7.349 µm to 82.334 µm, the prepared total mixed powder and tablets have good content uniformity, and the dissolution profiles of the formulations were basically consistent.

### Example 15-16

The composition of the formulation is the same as that of Example 10, and the preparation steps are the same as those in Examples 6 to 8. The uncoated tablets were prepared, and were film-coated with a film coating premix (gastric soluble). Dissolution profile was measured for the samples with different coating weight gains.

**Table 10. Dissolution profiles of tablets with different coating weight gain**

| Dissolution medium | Time (min) | Example 15 | Example 16 |
|---|---|---|---|
| | | Coating weight gain of about 2% | Coating weight gain of about 4% |
| | 5 | 47 | 48 |
| | 10 | 62 | 62 |
| pH6.8 buffer | 15 | 70 | 68 |
| | 30 | 73 | 71 |
| | 45 | 77 | 77 |
| | 60 | 80 | 79 |

Different coating weight gains within the study range have no significant influence on the dissolution profile of the tablets.

### Example 17

The samples of Example 8 and Example 15 were packed with dual-aluminum blisters (Polyamide/aluminum/polyvinyl chloride is cold-pressed into a solid pharmaceutical composite hard plate, pharmaceutical aluminum foil) or PVDC blister (polyvinyl chloride/polyvinylidene chloride, pharmaceutical aluminum foil) respectively, and then were placed under accelerated conditions (40°C/75%RH) for 6 months. The stability of the samples under different packaging conditions was investigated. The results are shown as follows. After 6 months of placement, the stability of the samples packed with dual-aluminum blisters was significantly better than that of the samples packed with PVDC blister.

**Table 11. Stability of samples in different package**

| Samples | Package | Day 0 | | Accelerated conditions for 6 months | |
|---|---|---|---|---|---|
| | | Max. amount of single impurity (%) | Total impurity (%) | Max. amount of single impurity (%) | Total impurity (%) |
| Sample of Example 8 | dual-aluminum blisters | 0.05 | 0.23 | 0.14 | 0.55 |
| Sample of Example 15 | dual-aluminum blisters | 0.06 | 0.24 | 0.17 | 0.53 |
| Sample of Example 15 | PVDC blisters | 0.06 | 0.24 | 0.38 | 1.1 |

### Example 18-19

The composition of the formulations is the same as that of Example 10. Compound A, mannitol, microcrystalline cellulose and cross-linked povidone were added into a wet mixing granulator, and the stirrer and shearing blades were turned on to mix the materials. After different mixing times, samples were taken to check the content uniformity of the mixture.

**Table 11. Content uniformity of samples after different mixing times**

| | | Example 18 | Example 19 |
|---|---|---|---|
| Mixing time (s) | | 180 | 270 |
| Content at each sampling points (%) | ① | 4.95 | 4.95 |
| | ② | 4.98 | 4.94 |
| | ③ | 4.99 | 5.02 |
| | ④ | 4.99 | 4.88 |
| | ⑤ | 5.04 | 4.97 |
| | ⑥ | 4.95 | 4.88 |
| | ⑦ | 4.96 | 5.07 |
| | ⑧ | 4.93 | 4.92 |
| | ⑨ | 5.07 | 4.83 |
| | ⑩ | 4.96 | 4.94 |
| Mean value (%) | | 4.98 | 4.94 |
| RSD (%) | | 0.9 | 1.5 |

Using a wet mixing granulator, the materials were homogeneously blended within a short processing time, achieving excellent mixing uniformity. Moreover, the mixing uniformity of the materials is good for different mixing times within the study range.

### Biological assay of Compound A

### 1. In Vitro DPP1 Enzyme Activity Assay

Recombinant human DPP1 enzyme (R&D Systems, Cat. No. 1071-CY) at a final concentration of 100 µg/mL was mixed with recombinant human cathepsin L (R&D Systems, Cat. No. 952-CY) at a final concentration of 20 µg/mL. The mixture was incubated at room temperature for 1 hour to activate the DPP1 enzyme. The activated DPP1 enzyme was diluted by 100-fold. To a 384-well plate 5 µL of compounds at different concentrations and 5 µL of the diluted DPP1 enzyme were added and incubated at room temperature for 30 minutes. After the addition of 10 µL of the substrate Gly-Arg-AMC (bachem, Cat. No. I-1215) at a concentration of 20 µM, incubation was continued at room temperature for 60 minutes. The fluorescence intensity was detected with a microplate reader with excitation at 380 nm and emission at 460 nm. IC₅₀ values were calculated using Origin2019 software with DosResp function.

Test results: the compound of the present invention showed inhibitory activity against DPP1 receptor. The compounds of the Examples has a IC₅₀ values of less than 100 nM against DPP1 receptor. The test result of some Examples was shown in Table 8.

**Table 8. Inhibitory activity against DPP1**

| Compound No | IC₅₀/nM |
|---|---|
| Compound A | 1.6 |

Conclusion: Compound A of the present invention shows high inhibitory activity against DPP1 receptor.

### 2. Pharmacokinetic Experiment in Rats

1.1 Test animals: Male SD rats, ca. 220 g, 6-8 weeks old, 6 rats/compound. The rats were purchased from CHENGDU DOSSY EXPERIMENTAL ANIMALS CO., LTD.

1.2 Test design: On the day of the test, 6 SD rats were randomly divided into groups according to body weight. The rats were fasted but had access to water for 12-14 hours one day before administration. The rats were fed 4 hours after administration.

**Table 9. Information for administration**

| Groups | Number | Information for administration | | | | | |
|---|---|---|---|---|---|---|---|
| | Male | Test compound | Dosage (mg/kg ) | Concentratio n (mg/mL) | Volume (mL/kg) | Sample | Mode |
| G1 | 3 | INS 1007 | 1 | 0.2 | 5 | Plasma | Intraven ous |
| G2 | 3 | Compound A | 1 | 0.2 | 5 | Plasma | |
| G3 | 3 | INS 1007 | 3 | 0.3 | 10 | Plasma | Oral gavage |
| G4 | 3 | Compound A | 3 | 0.3 | 10 | Plasma | |

Vehicle for intravenous administration: 5% DMA + 5% Solutol + 90% Saline; Vehicle for oral gavage: 0.5% MC; control compound INS1007 is namely Compound 2 in patent WO2015110826A1, and was prepared according to the method described in the patent.

Rats were anesthetized with isoflurane before and after administration. Blood (0.1 ml) was collected by orbital blood collection and placed in EDTAK2 centrifuge tube. The blood sample was centrifuged at 5000 rpm at 4°C for 10 min. The plasma was collected. Blood sampling time point for the intravenous administration group: 0 min, 5 min, 15 min, 30 min, 1 h, 2 h, 4 h, 6 h, 8 h, and 24 h; and blood sampling time point for oral gavage group: 0 min, 5 min, 15 min, 30 min, 1 h, 2 h, 4 h, 6 h, 8 h, and 24 h. All samples were stored at -80° C till analysis and detection.

**Table 10. Pharmacokinetic parameters of test compounds in rat plasma**

| Test compound | Administration Route | CL (mL/min/kg) | Vdss (L/kg) | AUC0-t (hr*ng/mL) | F (%) |
|---|---|---|---|---|---|
| INS 1007 | Intravenous (1 mg/kg) | 2.08 | 0.738 | 8396 | - |
| Compound A | | 1.66 | 0.753 | 9503 | - |
| INS 1007 | Oral gavage (3 mg/kg) | - | - | 22201 | 88.1 |
| Compound A | | - | - | 31159 | >100 |

Conclusion: The compound of the present invention has good bioavailability and pharmacokinetic profile.

### 3. Toxicity test of repeated oral administration for 14 days in rats

SD rats were randomly divided into the following groups according to body weight: vehicle control group (0.5% MC), INS1007 groups (30, 100 and 300 mg/kg), and Compound A groups (30, 100 and 300 mg/kg). The treatment group each includes 16 rats, and the vehicle control group includes 10 rats, with a half of male rats and a half of female rats. The drug at corresponding concentrations and the vehicle were administered to the rats through oral gavage daily for 14 consecutive days. The rats were allowed to recover for 7 days. During the administration period, general conditions, body weight and food intake were detected for each group. After the administration period and the recovery period, hematology test, serum biochemistry test and gross anatomical examination were performed for each group.

Conclusion: At the same dose, Compound A of the present invention are less toxic than INS1007 and has better safety.

## Claims

1. A pharmaceutical composition comprising Compound A and an excipient, wherein Compound A has the structure of

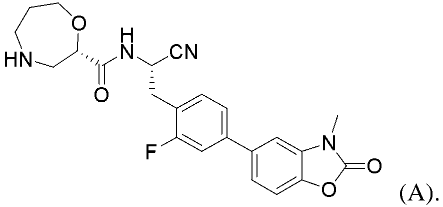

2. The pharmaceutical composition according to claim 1, wherein the amount of Compound A in the pharmaceutical composition is 1%-20%, preferably 2.5%-10%, more preferably 5%, by mass of the total composition.

3. The pharmaceutical composition according to claim 1 or 2, wherein the particle size distribution d(0.9) of Compound A is less than or equal to 150 µm, preferably less than or equal to 100 µm, more preferably less than or equal to 85 µm.

4. The pharmaceutical composition according to claim 3, wherein the processing method for Compound A is one or more of sieving, mechanical grinding, and air-flow grinding.

5. The pharmaceutical composition according to any one of claims 1-4, wherein the excipient comprises a filler which is selected from one or more of lactose, microcrystalline cellulose, sucrose, glucose, powdered cellulose, calcium phosphate, calcium hydrogen phosphate, calcium carbonate, aluminum silicate, dextrin, starch, pregelatinized starch, sodium chloride, potassium chloride, mannitol, or sorbitol, preferably one or more of lactose, microcrystalline cellulose, or mannitol.

6. The pharmaceutical composition according to claim 5, wherein the filler is mannitol and microcrystalline cellulose, and the mass ratio of mannitol to microcrystalline cellulose is from 3:1 to 1:1, preferably 1:1.

7. The pharmaceutical composition according to claim 5, wherein the filler is lactose and microcrystalline cellulose, and the mass ratio of lactose to microcrystalline cellulose is from 3:1 to 1:1, preferably 3:1.

8. The pharmaceutical composition according to any one of claims 5-7, wherein the excipient further comprises a disintegrant.

9. The pharmaceutical composition according to claim 8, wherein the disintegrant is one or more of starch, pregelatinized starch, sodium carboxymethyl starch, cross-linked povidone or croscarmellose sodium, preferably cross-linked povidone.

10. The pharmaceutical composition according to claim 9, wherein the amount of the cross-linked povidone is from 0.5% to 10%, preferably from 3% to 7%, more preferably 5%, by mass of the total composition.

11. The pharmaceutical composition according to any one of claims 8-10, wherein the excipient further comprises a lubricant.

12. The pharmaceutical composition according to claim 11, wherein the lubricant is one or more of stearic acid, magnesium stearate, calcium stearate, aluminum stearate, palmitic acid, glyceryl behenate, polyethylene glycols with various molecular weights, hydrogenated castor oil, and sodium stearyl fumarate, preferably magnesium stearate.

13. The pharmaceutical composition according to claim 12, wherein the amount of magnesium stearate is from 0.25% to 2%, preferably from 0.5% to 1.5%, more preferably 1%, by mass of the total composition.

14. The pharmaceutical composition according to any one of claims 5-13, further comprising a binder.

15. The pharmaceutical composition according to claim 14, wherein the binder is selected from one or more of starch slurry, povidone, hydroxypropyl cellulose, hydroxypropyl methylcellulose, ethyl cellulose, sodium carboxymethyl cellulose, xanthan gum, gum arabic, gelatin, guar gum or carbomer, preferably povidone, the amount of povidone being from 0.1% to 10%, preferably from 3% to 7%, more preferably 7%, by mass of the total composition.

16. The pharmaceutical composition according to any one of claims 1-15, wherein the dosage form of the pharmaceutical composition is tablet, capsule, granule or powders.

17. The pharmaceutical composition according to claim 16, wherein the dosage form of the pharmaceutical composition is tablet.

18. The pharmaceutical composition according to claim 17, wherein the tablets further comprise a film coating layer; the tablet is coated with a gastric soluble film coating premix or a coating material, wherein the coating materials is selected from one or more of polyvinyl alcohol, hydroxypropyl methylcellulose, talc, polyethylene glycol, polyethylene glycol-polyvinyl alcohol copolymer, titanium dioxide, glyceryl mono- and dicaprylocaprate, and lake; preferably, the tablet is coated with a gastric soluble film coating premix; wherein the target coating weight gain is from 0.5% to 10%, preferably from 1% to 6%, more preferably from 2% to 4%, by mass of the tablet core.

19. A pharmaceutical composition, wherein the pharmaceutical composition comprises Compound A, a filler, a lubricant, a disintegrant and coating material.

20. The pharmaceutical composition according to claim 19, wherein the amount of Compound A is 1%-20%, preferably 2.5%-10%, more preferably 5%, by mass of the total composition;
the amount of the filler is from 70% to 99%, preferably from 80% to 90%, more preferably 89%, by mass of the total composition;
the amount of the lubricant is from 0.25% to 2%, preferably from 0.5% to 1.5%, more preferably 1%, by mass of the total composition;
the amount of the disintegrant is from 0.5% to 10%, preferably from 3% to 7%, more preferably 5%, by mass of the total composition;
for the coating material, the target coating weight gain is from 0.5% to 10%, preferably from 1% to 6%, more preferably from 2% to 4%, by mass of the tablet core.

21. The pharmaceutical composition according to claim 19 or 20, wherein the filler is mannitol and microcrystalline cellulose, and the mass ratio of mannitol to microcrystalline cellulose is from 3:1 to 1:1, preferably 1:1; the lubricant is magnesium stearate; the disintegrant is cross-linked povidone; the coating material is gastric soluble film coating premix.

22. The pharmaceutical composition according to any one of claims 1-21, wherein the unit dosage of the formulation is selected from 1 mg to 500 mg, preferably 1 mg, 5 mg, 10 mg, 15 mg, 20 mg, 25 mg, 30 mg, 40 mg, 50 mg, 60 mg, 75 mg, 100 mg, 150 mg or 200 mg, calculated based on the mass of the active substance.

23. The pharmaceutical composition according to any one of claims 1-22, wherein the unit formulation of the pharmaceutical composition comprises 5 mg Compound A, 44.5 mg mannitol, 44.5 mg microcrystalline cellulose, 5 mg cross-linked povidone, 1 mg magnesium stearate, 3 mg coating powder; or the unit formulation of the pharmaceutical composition comprises 20 mg Compound A, 178 mg mannitol, 178 mg microcrystalline cellulose, 20 mg cross-linked povidone, 4 mg magnesium stearate, 12 mg coating powder.

24. A method for preparing the pharmaceutical composition according to any one of claims 1-23, wherein the preparation process is one or more of dry granulation process, wet granulation process, and direct powder compression process.

25. A method for preparing the pharmaceutical composition according to any one of claims 18-23, comprising the steps of:
(1) crushing Compound A;
(2) adding Compound A, a filler and a disintegrant into a mixer for premixing;
(3) adding a lubricant into the mixer and mixing thoroughly with the premixed powder; the mixture is compressed into a tablet, film-coated, and packaged.

26. The method for preparing according to claim 25, wherein the mixer includes one or more of: a mixer which mixes the materials by driving stirring blades or cutting knives at high speed, preferably a wet mixing granulator or a high-energy mixer, or a mixer which mixes the materials in a container by rotating the equipment container, preferably a three-dimensional mixer or a hopper mixer.

27. Use of the pharmaceutical composition according to any one of claims 1-23 in the manufacture of a medicament for treating diseases associated with fibrotic bronchiectasis.

28. The use according to claim 27, wherein the diseases associated with fibrotic bronchiectasis is selected from lower respiratory tract diseases caused by bronchiectasis, and acute lung injury or acute respiratory distress syndrome.
